# EUROPEAN PATENT SPECIFICATION

(11) **EP 2 517 018 B1**
(45) Date of publication and mention of the grant of the patent: **05.11.2014**
(21) Application number: 10798830.5
(22) Date of filing: 20.12.2010
(51) Int. Cl.: G01N 33/68

(54) **Biomarkers**
Biomarker
Biomarqueurs

(30) Priority: 21.12.2009 GB 0922236
(43) Date of publication of application: 31.10.2012
(60) Divisional application: 14186026.2
(73) Proprietor: Cambridge Enterprise Ltd., Cambridge Cambridgeshire CB2 1TN (GB)
(72) Inventor: BAHN, Sabine, Cambridge Cambridgeshire CB2 1QT (GB); SCHWARZ, Emanuel, Cambridge Cambridgeshire CB2 1QT (GB)
(74) Representative: Gibson, Mark
(86) International application number: PCT/GB2010/052160
(87) International publication number: WO 2011/077130

(56) References cited:
- WO-A1-2009/077763

## Description

### FIELD OF THE INVENTION

The invention relates to a method of diagnosing or monitoring schizophrenia or other psychotic disorder.

### BACKGROUND OF THE INVENTION

Schizophrenia is a psychiatric diagnosis that describes a mental disorder characterized by abnormalities in the perception or expression of reality. It most commonly manifests as auditory hallucinations, paranoid or bizarre delusions, or disorganized speech and thinking with significant social or occupational dysfunction. Onset of symptoms typically occurs in young adulthood, with approximately 0.4-0.6% of the population affected. Diagnosis is based on the patient's self-reported experiences and observed behavior. No laboratory test for schizophrenia currently exists.

Studies suggest that genetics, early environment, neurobiology, psychological and social processes are important contributory factors; some recreational and prescription drugs appear to cause or worsen symptoms. Current psychiatric research is focused on the role of neurobiology, but no single organic cause has been found. Due to the many possible combinations of symptoms, there is debate about whether the diagnosis represents a single disorder or a number of discrete syndromes.

The disorder is thought to mainly affect cognition, but it also usually contributes to chronic problems with behavior and emotion. People with schizophrenia are likely to have additional (comorbid) conditions, including major depression and anxiety disorders; the lifetime occurrence of substance abuse is around 40%. Social problems, such as long-term unemployment, poverty and homelessness, are common. Furthermore, the average life expectancy of people with the disorder is 10 to 12 years less than those without, due to increased physical health problems and a higher suicide rate.

An important utility of biomarkers for psychotic disorders is their response to medication. Administration of antipsychotics remains a subjective process, relying solely on the experience of clinicians. Furthermore, the development of antipsychotic drugs has been based on chance findings often with little relation to the background driving the observations.

Schizophrenia is treated primarily with antipsychotic medications which are also referred to as neuroleptic drugs or neuroleptics. Newer antipsychotic agents such as Clozapine, Olanzapine, Quetiapine or Risperidone are thought to be more effective in improving negative symptoms of psychotic disorders than older medication like Chlorpromazine. Furthermore, they induce less extrapyramidal side effects (EPS) which are movement disorders resulting from antipsychotic treatment.

The history of neuroleptics dates back to the late 19th century. The flourishing dye industry catalyzed development of new chemicals that lay the background to modern day atypical antipsychotics. Developments in anti malaria, antihistamine and anaesthetic compounds also produced various neuroleptics. The common phenomenon to all these processes is a fundamental lack of understanding of the biological mechanisms and pathways that these drugs affect, apart from the observation that they prominently block D2 receptors in the striatum.

There is therefore a pressing need for objective molecular readouts that can diagnose schizophrenia or other psychotic disorders and furthermore indicate whether a patient is responding to medication, as well as for predicting prognosis.

### SUMMARY OF THE INVENTION

According to a first aspect of the invention, there is provided the use of a1 antitrypsin, apolipoprotein H, complement 3, carcinoembryonic antigen, cortisol, connective tissue growth factor (CTGF), ferritin, haptoglobin, interleukin-10, macrophage inflammatory factor (MIF), prolactin, serum amyloid P and tissue inhibitor of metalloprotease-1 (TIMP 1) as a specific panel of analyte biomarkers for schizophrenia or predisposition thereto.

According to a further aspect of the invention, there is provided a method of diagnosing or monitoring schizophrenia, or predisposition thereto, comprising detecting and/or quantifying, in a sample from a test subject, the specific panel of analyte biomarkers defined herein.

According to a further aspect of the invention, there is provided a method of monitoring efficacy of a therapy in a subject having, suspected of having, or of being predisposed to schizophrenia, comprising detecting and/or quantifying, in a sample from said subject, the specific panel of analyte biomarkers defined herein.

Disclosed are ligands, such as naturally occurring or chemically synthesised compounds, capable of specific binding to the analyte biomarker. A ligand may comprise a peptide, an antibody or a fragment thereof, or an aptamer or oligonucleotide, capable of specific binding to the analyte biomarker. The antibody can be a monoclonal antibody or a fragment thereof capable of specific binding to the analyte biomarker. A ligand may be labelled with a detectable marker, such as a luminescent, fluorescent or radioactive marker; alternatively or additionally a ligand according to the invention may be labelled with an affinity tag, e.g. a biotin, avidin, streptavidin or His (e.g. hexa-His) tag.

A biosensor may comprise the analyte biomarker or a structural/shape mimic thereof capable of specific binding to an antibody against the analyte biomarker. Also provided is an array comprising a ligand or mimic as described herein.

Also provided is the use of one or more ligands as described herein, which may be naturally occurring or chemically synthesised, and is suitably a peptide, antibody or fragment thereof, aptamer or oligonucleotide, or the use of a biosensor of the invention, or an array of the invention, or a kit of the invention to detect and/or quantify the analyte. In these uses, the detection and/or quantification can be performed on a biological sample such as from the group consisting of CSF, whole blood, blood serum, plasma, urine, saliva, or other bodily fluid, breath, e.g. as condensed breath, or an extract or purification therefrom, or dilution thereof.

Diagnostic or monitoring kits are provided. Such kits will suitably comprise a ligand, for detection and/or quantification of the analyte biomarker, and/or a biosensor, and/or an array as described herein, optionally together with instructions for use of the kit.

A further aspect is a kit for monitoring or diagnosing schizophrenia or other psychotic disorder, comprising a biosensor capable of detecting and/or quantifying the specific panel of analyte biomarkers as defined herein.

Biomarkers for schizophrenia or other psychotic disorders are essential targets for discovery of novel targets and drug molecules that retard or halt progression of the disorder. As the level of the analyte biomarker is indicative of disorder and of drug response, the biomarker is useful for identification of novel therapeutic compounds in *in vitro* and/or *in vivo* assays. Biomarkers of the invention can be employed in methods for screening for compounds that modulate the activity of the analyte.

Thus, in a further aspect, there is provided the use of a ligand, as described, which can be a peptide, antibody or fragment thereof or aptamer or oligonucleotide; or the use of a biosensor, or an array; or a kit, to identify a substance capable of promoting and/or of suppressing the generation of the biomarker.

Also there is provided a method of identifying a substance capable of promoting or suppressing the generation of the analyte in a subject, comprising administering a test substance to a subject animal and detecting and/or quantifying the level of the analyte biomarker present in a test sample from the subject.

### BRIEF DESCRIPTION OF THE FIGURES

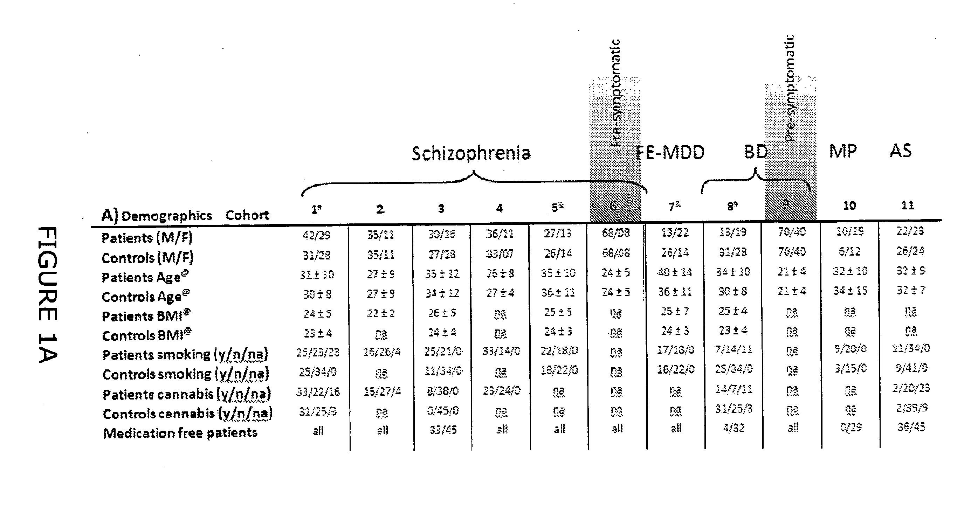

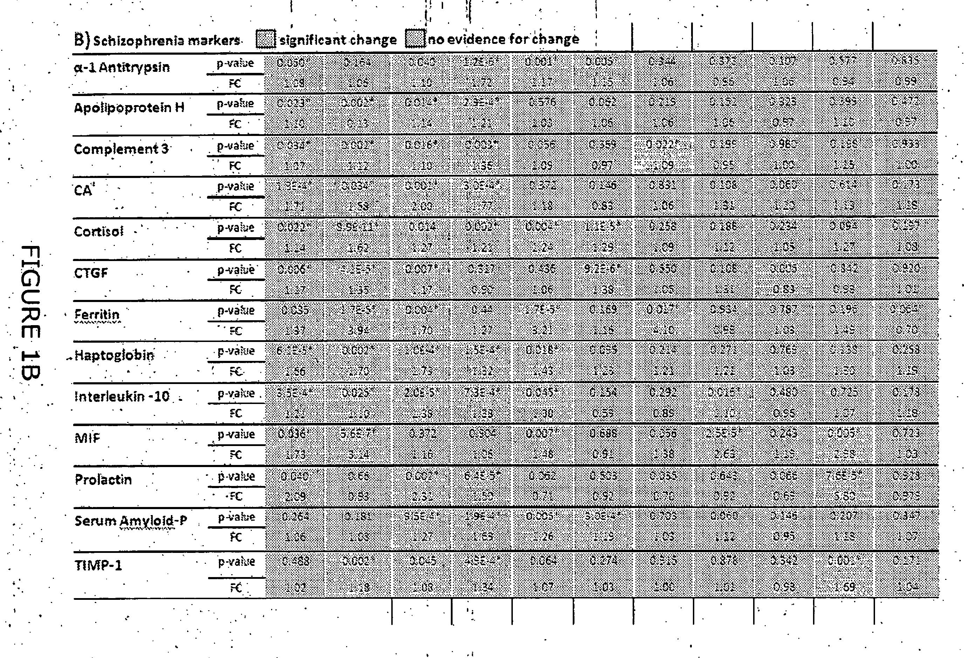

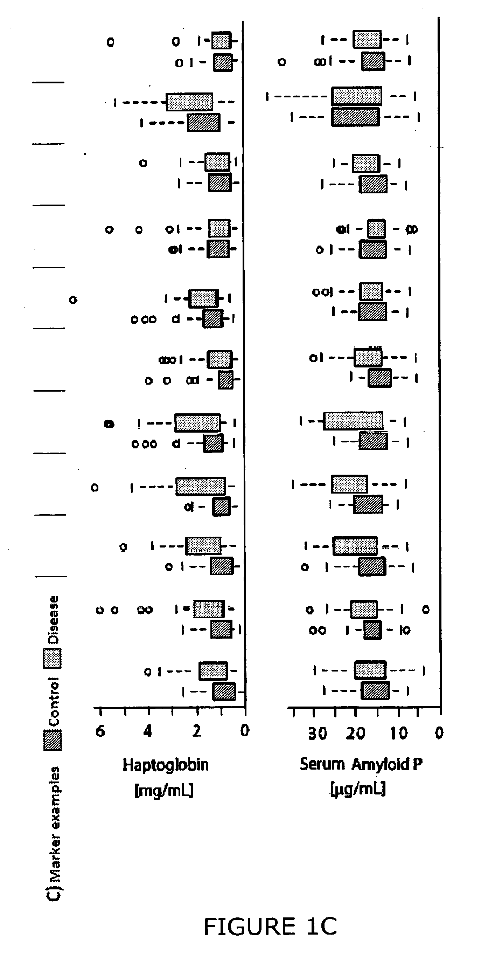

**FIGURE 1****:** HumanMap® profiling of clinical serum samples resulting in the identification of a molecular signature for schizophrenia. **A)** Demographic details. Cohorts 1-5: schizophrenia - paranoid subtype (295.30). Cohort 6: pre-symptomatic subjects later diagnosed with schizophrenia (295.1- 295.3, 295.6, 295.7, 295.9). Cohort 7: acutely ill major depressive disorder (FE-MDD). Cohort 8: bipolar disorder (BD) euthymic [types I (296.4) and II (296.89)]. Cohort 9: BD pre-symptomatic (296.00-296.06; 296.40-296.7, 296.89). Cohort 10: BD subjects in mania phase (manic psychosis; MP). Cohort 11: Asperger syndrome (AS). Subjects were matched for the indicated parameters and the medication status of each patient group is indicated. M/F = male/female; BMI = body mass index; Y/N = yes/no; na = not available. ^{@}Values are shown as mean ± sd. control groups of cohorts 1 and 8, and those of cohorts 5 and 7 were identical. **B)** Expression profiles of 13 serum analytes in schizophrenia compared to MDD, BD, MP and AS subjects. Significant differences (two-tailed parametric t-test; dark grey) and no change (pale grey) are shown for all patient and control comparisons. Measurements that were also significant using non-parametric Wilcoxon rank sum tests are indicated by an asterisk. CA = carcinoembryonic antigen, CTGF = connective tissue growth factor; MIF = macrophage migration inhibitory factor. Timp 1 = tissue inhibitor of metalloproteases 1. Prolactin was also changed in cohort 7, but the observed fold change was opposite to the one observed in the schizophrenia cohorts. **C)** Expression profile changes of serum amyloid P and haptoglobin in patient and control populations across the 11 cohorts. The expression levels are given as box plots for patients (pale grey) and controls (dark grey).

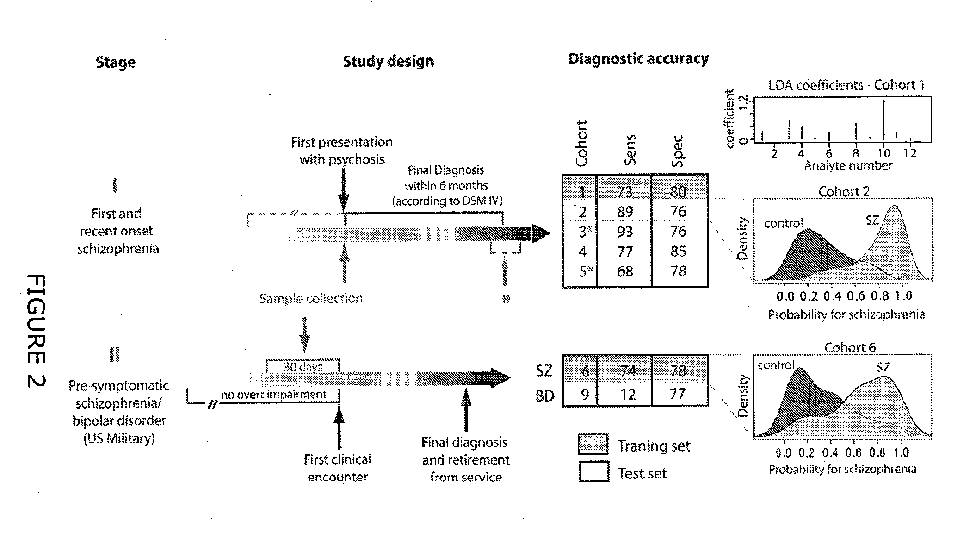

**FIGURE 2:** Diagnostic accuracy of the 13 analyte panel for schizophrenia. The study design indicates times of sera collection (dark arrows). Final DSM-IV diagnosis was made at first presentation or within 6 months after manifestation. Asterisks indicate samples collected before or after final diagnosis. Sensitivity and specificity values (%) were determined for distinguishing schizophrenia (SZ) or BD subjects from controls using Linear Discriminant Analysis. The algorithm was trained on cohort 1 and tested blindly on cohorts 2-5. The algorithm was also trained on the presymptomatic schizophrenia subjects in cohort 6 and compared to presymptomatic BD by blind testing on cohort 9. Sensitivity and specificity were estimated in cohort 6 using leave one out cross validation. Coefficients of the linear discriminants are shown for each analyte on the top right (algorithm built on cohort 1). The density distributions describe the output of the algorithm for cohort 2 (blinded prediction using the algorithm trained on cohort 1) and for cohort 6 (leave one out cross-validation estimate). The algorithm output ranged from 0-1 and was smoothed for illustration purposes. For the calculation of classification accuracy, a cut point of 0.5 was used.

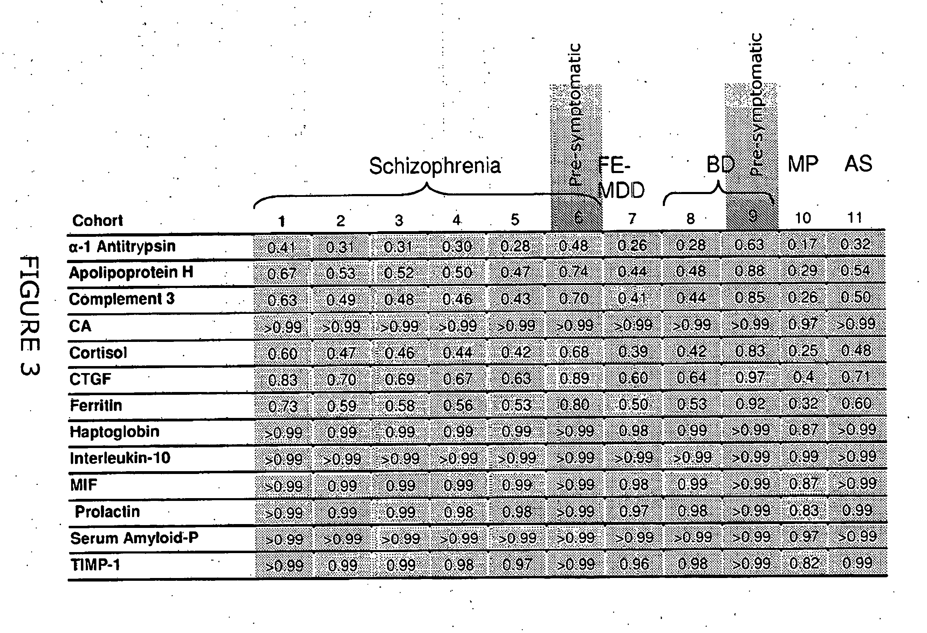

**FIGURE 3:** Power calculations for two-tailed T-tests (alpha=0.05, unequal group sizes) were performed based on the effect size in cohort 4 (serum amyloid P and TIMP-1) and cohort 1 (remaining analytes). Significant differences in analyte levels between patients and controls are indicated in dark grey. Non-significant differences are shown in pale grey.

### DETAILED DESCRIPTION OF THE INVENTION

The term "biomarker" means a distinctive biological or biologically derived indicator of a process, event, or condition. Analyte biomarkers can be used in methods of diagnosis, e.g. clinical screening, and prognosis assessment and in monitoring the results of therapy, identifying patients most likely to respond to a particular therapeutic treatment, drug screening and development. Biomarkers and uses thereof are valuable for identification of new drug treatments and for discovery of new targets for drug treatment.

It will be readily apparent to the skilled person that the analytes listed herein are known and have been described in the literature.

According to a first aspect of the invention, there is provided the use of α1 antitrypsin, apolipoprotein H, complement 3, carcinoembryonic antigen, cortisol, connective tissue growth factor (CTGF), ferritin, haptoglobin, interleukin-10, macrophage inflammatory factor (MIF), prolactin, serum amyloid P and tissue inhibitor of metalloprotease-1 (TIMP 1) as a specific panel of analyte biomarkers for schizophrenia or other psychotic disorder, or predisposition thereto.

The 13 analytes within the specific panel of analyte biomarkers of the invention have previously been disclosed in International Patent Application No. PCT/GB2008/004186 along with over 100 other analyte biomarkers for the diagnosis of psychotic disorders such as schizophrenia. The present invention relates to the identification of a specific and unique pattern of serum analyte biomarkers which can be used for diagnosis of schizophrenia or other psychotic disorder in a specific and sensitive manner. More particularly, a set of 13 differentially expressed analytes have identified an underlying biological signature in serum of first and recent onset schizophrenia subjects using the multiplex molecular profiling approach defined herein. This molecular signature was surprisingly replicated across five independent cohorts of schizophrenia patients and was also capable of identifying as yet unaffected individuals who later went on to develop schizophrenia. Importantly, this signature was not apparent in related psychiatric disorders such as major depressive disorder, bipolar disorder, manic psychosis and Asperger syndrome, suggesting that these findings hold promise for the future development of a rapid and non-invasive blood test to facilitate early or even pre-symptomatic diagnosis of schizophrenia. This would be an important breakthrough since it would aid clinicians to identify vulnerable patients early on in the disease process allowing for earlier or even preventative therapeutic intervention and better outcomes.

Therefore, the specific panel of 13 serum biomarkers provided by the invention is a sensitive and specific predictor for the presence of schizophrenia or other psychotic disorder.

References herein to "other psychotic disorder" relate to any appropriate psychotic disorder according to DSM-IV Diagnostic and Statistical Manual of Mental Disorders, 4th edition, American Psychiatric Assoc, Washington, D.C., 2000. In one particular embodiment, the other psychotic disorder is schizophrenia. Examples of psychotic disorders related to schizophrenia include brief psychotic disorder delusional disorder, psychotic disorder due to a general medical condition, schizoeffective disorder, schizophreniform disorder, and substance-induced psychotic disorder. Schizophrenia is suitably early onset schizophrenia or first onset schizophrenia.

As used herein, the term "biosensor" means anything capable of detecting the presence of the biomarker. Examples of biosensors are described herein.

Biosensors may comprise a ligand or ligands, as described herein, capable of specific binding to the analyte biomarker. Such biosensors are useful in detecting and/or quantifying an analyte of the invention. Diagnostic kits for the diagnosis and monitoring of schizophrenia or other psychotic disorder are described herein. The kits additionally contain a biosensor capable of detecting and/or quantifying an analyte biomarker.

Monitoring methods can be used to monitor onset, progression, stabilisation, amelioration and/or remission.

In methods of diagnosing or monitoring according to the invention, detecting and/or quantifying the analyte biomarker in a biological sample from a test subject may be performed on two or more occasions. Comparisons may be made between the level of biomarker in samples taken on two or more occasions. Assessment of any change in the level of the analyte biomarker in samples taken on two or more occasions may be performed. Modulation of the analyte biomarker level is useful as an indicator of the state of schizophrenia or other psychotic disorder or predisposition thereto. An increase in the level of the biomarker, over time is indicative of onset or progression, i.e. worsening of this disorder, whereas a decrease in the level of the analyte biomarker indicates amelioration or remission of the disorder, or vice versa.

A method of diagnosis or monitoring according to the invention may comprise quantifying the analyte biomarker in a test biological sample from a test subject and comparing the level of the analyte present in said test sample with one or more controls.

The control used in a method of the invention can be one or more control(s) selected from the group consisting of: the level of biomarker analyte found in a normal control sample from a normal subject, a normal biomarker analyte level; a normal biomarker analyte range, the level in a sample from a subject with schizophrenia or other psychotic disorder, or a diagnosed predisposition thereto; schizophrenia or other psychotic disorder biomarker analyte level, or schizophrenia or other psychotic disorder biomarker analyte range.

There is provided a method of diagnosing schizophrenia or other psychotic disorder, or predisposition thereto, which comprises:
(a) quantifying the amount of the analyte biomarker in a test biological sample; and
(b) comparing the amount of said analyte in said test sample with the amount present in a normal control biological sample from a normal subject.

A higher level of the analyte biomarker in the test sample relative to the level in the normal control is indicative of the presence of schizophrenia or other psychotic disorder, or predisposition thereto; an equivalent or lower level of the analyte in the test sample relative to the normal control is indicative of absence of schizophrenia or other psychotic disorder and/or absence of a predisposition thereto.

The term "diagnosis" as used herein encompasses identification, confirmation, and/or characterisation of schizophrenia or other psychotic disorder, or predisposition thereto. By predisposition it is meant that a subject does not currently present with the disorder, but is liable to be affected by the disorder in time. Methods of monitoring and of diagnosis according to the invention are useful to confirm the existence of a disorder, or predisposition thereto; to monitor development of the disorder by assessing onset and progression, or to assess amelioration or regression of the disorder. Methods of monitoring and of diagnosis are also useful in methods for assessment of clinical screening, prognosis, choice of therapy, evaluation of therapeutic benefit, i.e. for drug screening and drug development.

Efficient diagnosis and monitoring methods provide very powerful "patient solutions" with the potential for improved prognosis, by establishing the correct diagnosis, allowing rapid identification of the most appropriate treatment (thus lessening unnecessary exposure to harmful drug side effects), reducing relapse rates.

Also provided is a method of monitoring efficacy of a therapy for schizophrenia or other psychotic disorder in a subject having such a disorder, suspected of having such a disorder, or of being predisposed thereto, comprising detecting and/or quantifying the analyte present in a biological sample from said subject. In monitoring methods, test samples may be taken on two or more occasions. The method may further comprise comparing the level of the biomarker(s) present in the test sample with one or more control(s) and/or with one or more previous test sample(s) taken earlier from the same test subject, e.g. prior to commencement of therapy, and/or from the same test subject at an earlier stage of therapy. The method may comprise detecting a change in the level of the biomarker(s) in test samples taken on different occasions.

Provided is a method for monitoring efficacy of therapy for schizophrenia or other psychotic disorder in a subject, comprising:
(a) quantifying the amount of the analyte biomarker; and
(b) comparing the amount of said analyte in said test sample with the amount present in one or more control(s) and/or one or more previous test sample(s) taken at an earlier time from the same test subject.

A decrease in the level of the analyte biomarker in the test sample relative to the level in a previous test sample taken earlier from the same test subject is indicative of a beneficial effect, e.g. stabilisation or improvement, of said therapy on the disorder, suspected disorder or predisposition thereto.

Methods for monitoring efficacy of a therapy can be used to monitor the therapeutic effectiveness of existing therapies and new therapies in human subjects and in non-human animals (e.g. in animal models). These monitoring methods can be incorporated into screens for new drug substances and combinations of substances.

Suitably, the time elapsed between taking samples from a subject undergoing diagnosis or monitoring will be 3 days, 5 days, a week, two weeks, a month, 2 months, 3 months, 6 or 12 months. Samples may be taken prior to and/or during and/or following an anti-psychotic therapy. Samples can be taken at intervals over the remaining life, or a part thereof, of a subject.

The term "detecting" as used herein means confirming the presence of the analyte biomarker present in the sample. Quantifying the amount of the biomarker present in a sample may include determining the concentration of the analyte biomarker present in the sample. Detecting and/or quantifying may be performed directly on the sample, or indirectly on an extract therefrom, or on a dilution thereof.

In alternative aspects, the presence of the analyte biomarker is assessed by detecting and/or quantifying antibody or fragments thereof capable of specific binding to the biomarker that are generated by the subject's body in response to the analyte and thus are present in a biological sample from a subject having schizophrenia or other psychotic disorder or a predisposition thereto.

Detecting and/or quantifying can be performed by any method suitable to identify the presence and/or amount of a specific protein in a biological sample from a patient or a purification or extract of a biological sample or a dilution thereof. In methods, quantifying may be performed by measuring the concentration of the analyte biomarker in the sample or samples. Biological samples that may be tested in a method of the invention include cerebrospinal fluid (CSF), whole blood, blood serum, plasma, urine, saliva, or other bodily fluid (stool, tear fluid, synovial fluid, sputum), breath, e.g. as condensed breath, or an extract or purification therefrom, or dilution thereof. Biological samples also include tissue homogenates, tissue sections and biopsy specimens from a live subject, or taken post-mortem. The samples can be prepared, for example where appropriate diluted or concentrated, and stored in the usual manner.

Detection and/or quantification of analyte biomarkers may be performed by detection of the analyte biomarker or of a fragment thereof, e.g. a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length.

The biomarker may be directly detected, e.g. by SELDI or MALDI-TOF. Alternatively, the biomarker may be detected directly or indirectly via interaction with a ligand or ligands such as an antibody or a biomarker-binding fragment thereof, or other peptide, or ligand, e.g. aptamer, or oligonucleotide, capable of specifically binding the biomarker. The ligand may possess a detectable label, such as a luminescent, fluorescent or radioactive label, and/or an affinity tag.

For example, detecting and/or quantifying can be performed by one or more method(s) selected from the group consisting of: SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC and other LC or LC MS-based techniques. Appropriate LC MS techniques include ICAT® (Applied Biosystems, CA, USA), or iTRAQ® (Applied Biosystems, CA, USA). Liquid chromatography (e.g. high pressure liquid chromatography (HPLC) or low pressure liquid chromatography (LPLC)), thin-layer chromatography, NMR (nuclear magnetic resonance) spectroscopy could also be used.

Methods of diagnosing or monitoring may comprise analysing a sample of cerebrospinal fluid (CSF) by SELDI TOF or MALDI TOF to detect the presence or level of the analyte biomarker. These methods are also suitable for clinical screening, prognosis, monitoring the results of therapy, identifying patients most likely to respond to a particular therapeutic treatment, for drug screening and development, and identification of new targets for drug treatment.

Detecting and/or quantifying the analyte biomarkers may be performed using an immunological method, involving an antibody, or a fragment thereof capable of specific binding to the analyte biomarker. Suitable immunological methods include sandwich immunoassays, such as sandwich ELISA, in which the detection of the analyte biomarkers is performed using two antibodies which recognize different epitopes on a analyte biomarker; radioimmunoassays (RIA), direct, indirect or competitive enzyme linked immunosorbent assays (ELISA), enzyme immunoassays (EIA), Fluorescence immunoassays (FIA), western blotting, immunoprecipitation and any particle-based immunoassay (e.g. using gold, silver, or latex particles, magnetic particles, or Q-dots). Immunological methods may be performed, for example, in microtitre plate or strip format.

Immunological methods may be based, for example, on any of the following methods.

Immunoprecipitation is the simplest immunoassay method; this measures the quantity of precipitate, which forms after the reagent antibody has incubated with the sample and reacted with the target antigen present therein to form an insoluble aggregate. Immunoprecipitation reactions may be qualitative or quantitative.

In particle immunoassays, several antibodies are linked to the particle, and the particle is able to bind many antigen molecules simultaneously. This greatly accelerates the speed of the visible reaction. This allows rapid and sensitive detection of the biomarker.

In immunonephelometry, the interaction of an antibody and target antigen on the biomarker results in the formation of immune complexes that are too small to precipitate. However, these complexes will scatter incident light and this can be measured using a nephelometer. The antigen, i.e. biomarker, concentration can be determined within minutes of the reaction.

Radioimmunoassay (RIA) methods employ radioactive isotopes such as I¹²⁵ to label either the antigen or antibody. The isotope used emits gamma rays, which are usually measured following removal of unbound (free) radiolabel. The major advantages of RIA, compared with other immunoassays, are higher sensitivity, easy signal detection, and well-established, rapid assays. The major disadvantages are the health and safety risks posed by the use of radiation and the time and expense associated with maintaining a licensed radiation safety and disposal program. For this reason, RIA has been largely replaced in routine clinical laboratory practice by enzyme immunoassays.

Enzyme (EIA) immunoassays were developed as an alternative to radioimmunoassays (RIA). These methods use an enzyme to label either the antibody or target antigen. The sensitivity of EIA approaches that for RIA, without the danger posed by radioactive isotopes. One of the most widely used EIA methods for detection is the enzyme-linked immunosorbent assay (ELISA). ELISA methods may use two antibodies one of which is specific for the target antigen and the other of which is coupled to an enzyme, addition of the substrate for the enzyme results in production of a chemiluminescent or fluorescent signal.

Fluorescent immunoassay (FIA) refers to immunoassays which utilize a fluorescent label or an enzyme label which acts on the substrate to form a fluorescent product. Fluorescent measurements are inherently more sensitive than colorimetric (spectrophotometric) measurements. Therefore, FIA methods have greater analytical sensitivity than EIA methods, which employ absorbance (optical density) measurement.

Chemiluminescent immunoassays utilize a chemiluminescent label, which produces light when excited by chemical energy; the emissions are measured using a light detector.

Immunological methods can thus be performed using well-known methods. Any direct (e.g., using a sensor chip) or indirect procedure may be used in the detection of analyte biomarkers.

The Biotin-Avidin or Biotin-Streptavidin systems are generic labelling systems that can be adapted for use in immunological methods of the invention. One binding partner (hapten, antigen, ligand, aptamer, antibody, enzyme etc) is labelled with biotin and the other partner (surface, e.g. well, bead, sensor etc) is labelled with avidin or streptavidin. This is conventional technology for immunoassays, gene probe assays and (bio)sensors, but is an indirect immobilisation route rather than a direct one. For example a biotinylated ligand (e.g. antibody or aptamer) specific for an analyte biomarker may be immobilised on an avidin or streptavidin surface, the immobilised ligand may then be exposed to a sample containing or suspected of containing the analyte biomarker in order to detect and/or quantify an analyte biomarker. Detection and/or quantification of the immobilised antigen may then be performed by an immunological method as described herein.

The term "antibody" as used herein includes, but is not limited to: polyclonal, monoclonal, bispecific, humanised or chimeric antibodies, single chain antibodies, Fab fragments and F(ab')₂ fragments, fragments produced by a Fab expression library, anti-idiotypic (anti-Id) antibodies and epitope-binding fragments of any of the above. The term "antibody" as used herein also refers to immunoglobulin molecules and immunologically-active portions of immunoglobulin molecules, i.e., molecules that contain an antigen binding site that specifically binds an antigen. The immunoglobulin molecules can be of any class (e. g., IgG, IgE, IgM, IgD and IgA) or subclass of immunoglobulin molecule.

The identification of key biomarkers specific to a disease is central to integration of diagnostic procedures and therapeutic regimes. Using predictive biomarkers appropriate diagnostic tools such as biosensors can be developed; accordingly, in methods and uses, detecting and quantifying can be performed using a biosensor, microanalytical system, microengineered system, microseparation system, immunochromatography system or other suitable analytical devices. The biosensor may incorporate an immunological method for detection of the biomarker(s), electrical, thermal, magnetic, optical (e.g. hologram) or acoustic technologies. Using such biosensors, it is possible to detect the target biomarker(s) at the anticipated concentrations found in biological samples.

Thus, according to a further aspect there is provided an apparatus for diagnosing or monitoring schizophrenia or other psychotic disorders which comprises a biosensor, microanalytical, microengineered, microseparation and/or immunochromatography system configured to detect and/or quantify any of the analyte biomarkers defined herein.

The biomarker(s) can be detected using a biosensor incorporating technologies based on "smart" holograms, or high frequency acoustic systems, such systems are particularly amenable to "bar code" or array configurations.

In smart hologram sensors (Smart Holograms Ltd, Cambridge, UK), a holographic image is stored in a thin polymer film that is sensitised to react specifically with the biomarker. On exposure, the biomarker reacts with the polymer leading to an alteration in the image displayed by the hologram. The test result read-out can be a change in the optical brightness, image, colour and/or position of the image. For qualitative and semi-quantitative applications, a sensor hologram can be read by eye, thus removing the need for detection equipment. A simple colour sensor can be used to read the signal when quantitative measurements are required. Opacity or colour of the sample does not interfere with operation of the sensor. The format of the sensor allows multiplexing for simultaneous detection of several substances. Reversible and irreversible sensors can be designed to meet different requirements, and continuous monitoring of a particular biomarker of interest is feasible.

Suitably, biosensors for detection of one or more biomarkers combine biomolecular recognition with appropriate means to convert detection of the presence, or quantitation, of the biomarker in the sample into a signal. Biosensors can be adapted for "alternate site" diagnostic testing, e.g. in the ward, outpatients' department, surgery, home, field and workplace.

Biosensors to detect the specific panel of biomarkers of the invention include acoustic, plasmon resonance, holographic and microengineered sensors. Imprinted recognition elements, thin film transistor technology, magnetic acoustic resonator devices and other novel acousto-electrical systems may be employed in biosensors for detection of the specific panel of biomarkers of the invention.

Methods involving detection and/or quantification of the specific panel of analyte biomarkers of the invention can be performed on bench-top instruments, or can be incorporated onto disposable, diagnostic or monitoring platforms that can be used in a non-laboratory environment, e.g. in the physician's office or at the patient's bedside. Suitable biosensors for performing methods of the invention include "credit" cards with optical or acoustic readers. Biosensors can be configured to allow the data collected to be electronically transmitted to the physician for interpretation and thus can form the basis for e-neuromedicine.

Any suitable animal may be used as a subject non-human animal, for example a non-human primate, horse, cow, pig, goat, sheep, dog, cat, fish, rodent, e.g. guinea pig, rat or mouse; insect (e.g. *Drosophila*), amphibian (e.g. *Xenopus*) or *C. elegans.*

The test substance can be a known chemical or pharmaceutical substance, such as, but not limited to, an anti-psychotic disorder therapeutic; or the test substance can be novel synthetic or natural chemical entity, or a combination of two or more of the aforesaid substances.

There is provided a method of identifying a substance capable of promoting or suppressing the generation of the analyte biomarker in a subject, comprising exposing a test cell to a test substance and monitoring the level of the analyte biomarker within said test cell, or secreted by said test cell.

The test cell could be prokaryotic, however a eukaryotic cell will suitably be employed in cell-based testing methods. Suitably, the eukaryotic cell is a yeast cell, insect cell, *Drosophila* cell, amphibian cell (e.g. from *Xenopus*), *C. elegans* cell or is a cell of human, non-human primate, equine, bovine, porcine, caprine, ovine, canine, feline, piscine, rodent or murine origin.

In methods for identifying substances of potential therapeutic use, non-human animals or cells can be used that are capable of expressing the analyte.

Screening methods also encompass a method of identifying a ligand capable of binding to the analyte biomarker, comprising incubating a test substance in the presence of the analyte biomarker in conditions appropriate for binding, and detecting and/or quantifying binding of the analyte to said test substance.

High-throughput screening technologies based on the biomarker, uses and methods of the invention, e.g. configured in an array format, are suitable to monitor biomarker signatures for the identification of potentially useful therapeutic compounds, e.g. ligands such as natural compounds, synthetic chemical compounds (e.g. from combinatorial libraries), peptides, monoclonal or polyclonal antibodies or fragments thereof, which may be capable of binding the biomarker.

Methods can be performed in array format, e.g. on a chip, or as a multiwell array. Methods can be adapted into platforms for single tests, or multiple identical or multiple non-identical tests, and can be performed in high throughput format. Methods of the invention may comprise performing one or more additional, different tests to confirm or exclude diagnosis, and/or to further characterise a condition.

Further provided is a substance, e.g. a ligand, identified or identifiable by an identification or screening method or use. Such substances may be capable of inhibiting, directly or indirectly, the activity of the analyte biomarker, or of suppressing generation of the analyte biomarker. The term "substances" includes substances that do not directly bind the analyte biomarker and directly modulate a function, but instead indirectly modulate a function of the analyte biomarker. Ligands are also included in the term substances; ligands of the invention (e.g. a natural or synthetic chemical compound, peptide, aptamer, oligonucleotide, antibody or antibody fragment) are capable of binding, suitably specific binding, to the analyte.

Further provided is a substance for use in the treatment of schizophrenia or other psychotic disorder, or predisposition thereto.

Also provided is the use of a substance in the treatment of schizophrenia or other psychotic disorder, or predisposition thereto.

Also provided is the use of a substance as a medicament.

Yet further provided is the use of a substance in the manufacture of a medicament for the treatment of schizophrenia or other psychotic disorder, or predisposition thereto.

A kit for diagnosing or monitoring schizophrenia or other psychotic disorder, or predisposition thereto is provided. Suitably a kit according to the invention may contain one or more components selected from the group: a ligand specific for the analyte biomarker or a structural/shape mimic of the analyte biomarker, one or more controls, one or more reagents and one or more consumables; optionally together with instructions for use of the kit in accordance with any of the methods defined herein.

The identification of biomarkers for schizophrenia or other psychotic disorder permits integration of diagnostic procedures and therapeutic regimes. Currently there are significant delays in determining effective treatment and hitherto it has not been possible to perform rapid assessment of drug response. Traditionally, many anti-psychotic therapies have required treatment trials lasting weeks to months for a given therapeutic approach. Detection of an analyte biomarker can be used to screen subjects prior to their participation in clinical trials. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized brain therapies, as assessment of response can be used to fine-tune dosage, minimise the number of prescribed medications, reduce the delay in attaining effective therapy and avoid adverse drug reactions. Thus by monitoring a biomarker, patient care can be tailored precisely to match the needs determined by the disorder and the pharmacogenomic profile of the patient, the biomarker can thus be used to titrate the optimal dose, predict a positive therapeutic response and identify those patients at high risk of severe side effects.

Biomarker-based tests provide a first line assessment of 'new' patients, and provide objective measures for accurate and rapid diagnosis, in a time frame and with precision, not achievable using the current subjective measures.

Furthermore, diagnostic biomarker tests are useful to identify family members or patients at high risk of developing schizophrenia or other psychotic disorder. This permits initiation of appropriate therapy, or preventive measures, e.g. managing risk factors. These approaches are recognised to improve outcome and may prevent overt onset of the disorder.

Biomarker monitoring methods, biosensors and kits are also vital as patient monitoring tools, to enable the physician to determine whether relapse is due to worsening of the disorder, poor patient compliance or substance abuse. If pharmacological treatment is assessed to be inadequate, then therapy can be reinstated or increased; a change in therapy can be given if appropriate. As the biomarkers are sensitive to the state of the disorder, they provide an indication of the impact of drug therapy or of substance abuse.

The following study illustrates the invention.

### METHODS

### Clinical samples

The institutional ethical committees approved the protocols of the study, informed written consent was given by all participants and studies were conducted according to the Declaration of Helsinki. All diagnoses (DSM-IV) and clinical tests were performed by psychiatrists under Good Clinical Practice-compliance to minimize variability. Cohorts 1 and 8 were from the University of Cologne (Germany), cohort 2 from the University of Muenster (Germany), cohorts 3, 5 and 7 from the University of Magdeburg (Germany), cohort 4 from Erasmus University (Netherlands), cohorts 6 and 9 from the United States military (facilitated by the Stanley Medical Research Institute, MD, USA), cohort 10 from the Sheppard Pratt hospital (Baltimore, USA) and cohort 11 from the Department of Psychiatry, University of Cambridge (UK). Controls were recruited from the same geographical areas or institutes and matched to the respective patient populations as indicated. Blood samples were collected from all subjects into S-Monovette 7.5mL serum tubes (Sarstedt; Numbrecht, Germany) and serum prepared and stored at -80°C in Low Binding Eppendorf tubes (Hamburg, Germany).

### Multiplexed immunoassay

Approximately 180 analytes were measured in sera using the HumanMAP^{®} multiplexed antigen immunoassays in a CLIA-certified laboratory at Rules Based Medicine. Assays were calibrated using standards, raw intensity measurements converted to absolute protein concentrations, and performance verified using quality control samples. Data analyses were performed using the statistical software package R (http://www.r-project.org). The protocol for the study participants, clinical samples and test methods was carried out in compliance with the Standards for Reporting of Diagnostic Accuracy (STARD) initiative (Bossuyt PM *et al* Standards for Reporting of Diagnostic Accuracy. *Clin Chem* 2003 Jan; **49**(1): 1-6).

### RESULTS

This study attempted to identify serum biomarkers which have proven crucial for aiding clinical diagnosis and effective treatment of many disorders but are still lacking for psychiatric illnesses such as schizophrenia (Amur S *et al Biomakers Med* 2008; **2**(3): 305-311). One reason for this is that it is not known whether psychiatric disorders can be identified by a biological signal in the peripheral circulation. The recent introduction of fluorescent bead-based technologies allows the simultaneous measurement of multiple analytes in small-volume samples, revolutionizing these analyses. Such analytical platforms are also suitable for further development of rapid, sensitive and specific diagnostic assays. With this in mind, the HumanMAP^{®} Multi-Analyte Profiling platform was used in collaboration with Rules Based Medicine (Austin, TX, USA) to analyze serum samples from 1061 individuals comprised of 326 first, recent onset and pre-symptomatic schizophrenia, 206 affective disorder (35 first episode major depressive disorder (MDD), 142 bipolar disorder (BD), 29 manic psychosis), 45 Asperger syndrome and 484 control subjects, recruited from psychiatric centres in Germany, Holland, the United Kingdom and the United States.

The HumanMAP^{®} technology has been shown to be reproducible and robust and has already been applied successfully in numerous clinical studies or biomarker discovery projects of diseases such as epithelial ovarian cancer (Bertenshaw GP et al Cancer Epidemiol Biomarkers Prev 2008 Oct; 17(10): 2872-2881), coronary artery disease (Gurbel PA et al Am Heart J 2008 Jan; 155(1): 56-61), myocardial infarction (Escobar GP, Lindsey ML. FASEB J 2007; 21(746.11)) and autoimmune disorders (Delaleu N et al Arthritis Res Ther 2008; 10(1): R22).

The first stage of this study was aimed at identifying a reproducible pattern of molecular changes in schizophrenia (n=250) compared to control subjects (n=230) across 5 independent cohorts (Figure 1A). Analytes were selected for the final panel based on biological reproducibility. This required that they had two-tailed p-values less than 0.05 in 3 or more cohorts and showed consistent directional changes. Cohorts 1, 2 and 4 were comprised of first onset antipsychotic-naïve subjects and most patients from cohort 3 and all patients from cohort 5 were antipsychotic-naïve or had been off medication for at least six weeks prior to sample collection. The remaining 12 patients from cohort 3 were medicated. All cohorts were matched for age and gender and only subjects with no medical co-morbidities or substance abuse were included. Cohort 1 was also matched for body mass index, smoking, cannabis consumption and date of sample collection and some of these parameters were also matched across the other cohorts (Figure 1A).

Analysis using the HumanMAP® platform resulted in identification of a signature consisting of 13 analytes using the above criteria (Figure 1B). At least 7 of these markers were altered consistently across all schizophrenia cohorts. An algorithm comprising the 13 analytes was trained on data from cohort 1 and tested blindly across cohorts 2-5, yielding an unweighted average sensitivity of 0.82 and specificity of 0.79 (Figure 2). It was also assessed whether there was any effect on biomarker profiles from any of the recorded demographic parameters. This analysis showed that only 14% of the observed changes in cohorts 1-5 were affected, suggesting that potential confounding factors did not influence the results significantly.

For the second stage of the study, serum samples were tested (cohort 6) collected by the United States military within one month of schizophrenic subjects first coming to the attention of clinical psychiatrists. As such, these individuals were also antipsychotic naïve. It should be noted that these samples were selected from a bank comprising approximately 43 million sera, which facilitated excellent matching for age, gender, ethnicity and lifestyle between diseased and control subjects. The use of illicit drugs was not a factor due to regular military screening procedures and, as with the other cohorts, only those individuals with no co-morbid medical histories were included in the study. HumanMAP® profiling showed that α1 antitrypsin, cortisol, connective tissue growth factor and serum amyloid P were altered significantly in these subjects (Figure 1B). The finding that these markers were present before overt clinical presentation of the disorder suggests that they could represent trait or early state markers for schizophrenia, whereas other analytes from the panel may represent state markers since these were present during the acute stage of the illness.

One factor which renders diagnosis of schizophrenia difficult is the overlap of symptoms with other neuropsychiatric disorders. For this reason, the 13 analyte panel was also tested using sera from 35 MDD (cohort 7), 142 BD (cohorts 8 and 9), 29 manic psychosis (cohort 10), 45 AS (cohort 11) and 277 control subjects. MDD subjects were acutely ill, drug naïve (n=22) or drug free (n=13) for at least 6 weeks prior to sample collection, and chosen due to the overlap of negative symptoms between depression and schizophrenia (Fleischhacker W. Encephale 2000 Oct; 26 Spec No 1: 12-14). Of the 32 euthymic BD patients (cohort 8), 28 were medicated and chosen for this study as such patients can experience disruptions in cognitive behaviours as seen in schizophrenia (Ferrier IN et al Br J Psychiatry 1999 Sep; 175: 246-251). The 110 BD patients (cohort 9) were medication-naïve and, similar to cohort 6, collected through the United States military before coming to the attention of clinical psychiatrists. Mania subjects were all medicated and selected for the study as they can display behaviours similar to the psychotic symptoms of schizophrenia (Dunayevich E, Keck PE, Jr. Curr Psychiatry Rep 2000 Aug; 2(4): 286-290). AS subjects were mostly un-medicated (36 out of 45 individuals) and were analyzed since they also overlap with schizophrenia for such symptoms as emotional lability, anxiety and poor social functioning (Raja M, Azzoni A. Gen Hosp Psychiatry 2001 Sep-Oct; 23(5): 285-293). The results of the HumanMAP® analysis showed that the biological signature was lower, with only 0 to 3 analytes altered in the non-schizophrenia cohorts (Figure 1B). Interestingly, the presymptomatic BD subjects (cohort 9) showed a significant alteration in only one of the analytes, suggesting that the panel was specific for schizophrenia. A power analysis was also performed to investigate potential effects of group size on ability to detect significant analyte differences between patients and controls in each cohort. This analysis revealed that for all 13 analytes, the statistical power was similar across all cohorts adding further support to the specificity of the results (Figure 3). An example of the biological profile of two of the analytes (haptoglobin and serum amyloid P) is shown across all 11 cohorts (Figure 1C). This illustrates the biological specificity of the signal for schizophrenia which is not apparent for the non-schizophrenia conditions.

To gain further insight into the differential diagnostic capability of the 13 analyte signature, an algorithm was trained on data from cohort 6 (schizophrenia) and this was tested on cohort 9 (BD). The two military cohorts were selected for this comparison as the subjects were matched closely across all parameters. Testing the algorithm on cohort 6 identified 56 out of the 76 schizophrenia subjects (sensitivity 0.74) and 60 of 76 controls (specificity 0.79) correctly (Figure 2). The accuracy of the signature for schizophrenia was shown by the finding that only 13 of the 110 BD patients (sensitivity = 0.12) had a signature similar to schizophrenia, and 85 of the 110 controls (specificity = 0.79) were identified correctly (Figure 2). To rule out any gender effects, the algorithm was retrained and retested using data from male subjects only. This gave essentially the same results with a good sensitivity for schizophrenia (sensitivity 0.75, specificity 0.79) and low sensitivity for this signal in BD subjects (sensitivity 0.11, specificity 0.77).

To investigate whether the 13 analyte signature was comprised of trait and/or state biomarkers, the performance of the panel was tested using samples from concordant (n=26) and discordant (n=36) twins for schizophrenia (data not shown). Partial Least Squares analysis showed that 75% of all affected twins clustered with schizophrenia subjects, consistent with the results of cohorts 1-5. Two subjects from discordant pairs who were unaffected at the time of sample collection and later developed schizophrenia, also showed a schizophrenia-like profile, as seen for cohort 6. However, only 22% of the remaining unaffected twins of discordant pairs showed a pattern similar to the schizophrenia signature, suggesting that genetic predisposition alone is not sufficient to cause significant serum abnormalities. To test this further, samples were analyzed which had been collected from patients after short term treatment with antipsychotics that resulted in improved symptoms (data not shown). Interesting, 60% of these subjects showed a shift from the schizophrenia signature to a more control-like pattern, suggesting that a significant proportion of the panel may contain biomarkers which reflect the state of the disorder.

All of the analytes identified as schizophrenia biomarkers have also been implicated in acute or chronic inflammatory conditions such as systemic lupus erythematosus (SLE). This is intriguing as around 22% of SLE patients also show a variety of neuropsychiatric symptoms similar to those in schizophrenia (Fessel WJ, Solomon GF. Calif Med 1960 Apr; 92: 266-270). This link is thought to arise from common inflammatory abnormalities in brain microvasculature, resulting in blood-brain barrier disturbances (Johnson RT, Richardson EP. Medicine (Baltimore) 1968 Jul; 47(4): 337-369; Bresnihan B et al Clin Exp Immunol 1977 Dec; 30(3): 333-337). However, it should be stressed that there are several markers on the HumanMAP® panel which have been associated previously with SLE (IL-2, IL-4, IL-6, TNF-alpha, MMP-9, and others) which showed no significant alterations in the present schizophrenia cohorts. This suggests that schizophrenia and SLE may share some aspects of an inflammatory component. Consistent with this, analysis of the 13 analytes *in silico* using the Ingenuity Pathways Knowledge Base (www.ingenuity.com) confirmed that the most significant functional pathway was "inflammatory response" (p=5.04E-9 - 4.10E-3). An altered inflammatory response has been associated with a number of other psychiatric disorders, as has dysregulation of the adrenal cortex hormone cortisol (Rybakowski JK, Wykretowicz A, Heymann-Szlachcinska A, Wysocki H. Impairment of endothelial function in unipolar and bipolar depression. Biol Psychiatry 2006 Oct 15; 60(8): 889-891). It was interesting in this regard that cortisol was elevated significantly across all schizophrenia cohorts and showed a trend for increase across all other non-schizophrenia cohorts (p=0.094-0.298).

Hypercortisolemia and hypothalamic-pituitary-adrenal hyperactivity may also be linked to the observed increase in macrophage migration inhibitory factor (MIF) levels. MIF has been shown to play a central role in the progression of immunological disturbances of SLE associated with atherosclerotic plaque development (Santos LL, Morand EF. Clin Chim Acta 2009 Jan; 399(1-2): 1-7; Burger-Kentischer A et al Circulation 2002 Apr 2; 105(13): 1561-1566). SLE and schizophrenia also share an increased prevalence of insulin resistance, metabolic syndrome and type II diabetes (Chung CP et al Ann Rheum Dis 2007 Feb; 66(2): 208-214; Wajed J et al Rheumatology (Oxford) 2004 Jan; 43(1): 7-12; De Hert M et al. Clin Pract Epidemol Ment Health 2006; 2: 14; Shoelson SE et al J Clin Invest 2006 Jul; 116(7): 1793-1801). This is consistent with previous findings of such abnormalities in the brain vasculature of patients and changes in glucoregulation in the schizophrenia brain and periphery (Guest P et al Molecular psychiatry (in press*);* Prabakaran S et al. Mol Psychiatry 2004 Jul; 9(7): 684-697, 643; Harris LW et al. PLoS One 2008; 3(12): e3964).

Further support of an inflammatory component in schizophrenia has been demonstrated by epidemiological studies which found that history of autoimmune disease was associated with a 45% increased risk for schizophrenia (Eaton WW et al. Am J Psychiatry 2006 Mar; 163(3): 521-528). A recent study published in *Nature* has shown that there is a significant association of single nucleotide polymorphisms and copy number variation within the major histocompatibility region, with an increased schizophrenia risk (Stefansson H et al. Nature 2009 Aug 6; 460(7256): 744-747). In addition, inflammation-related gene products have been shown to be altered in schizophrenia post-mortem brain studies and in plasma of living schizophrenia patients (Saetre P et al BMC Psychiatry 2007; 7: 46; Potvin S et al Biol Psychiatry 2008 Apr 15; 63(8): 801-808). Taken together, these findings suggest that inflammation may be a converging pathophysiological process in schizophrenia and other psychiatric and non-psychiatric disorders such as metabolic syndrome (Steiner J et al. Biol Psychiatry 2009 Jun 15; 65(12): 1107-1110). The biomarker signature comprising inflammatory biomarkers, identified in accordance with the present invention, already shows promise for distinguishing schizophrenia from controls and other psychiatric disorders. Therefore, further expansion of the signature by targeting hormonal, metabolic and neurotrophic pathways in larger population studies, may lead to a more robust diagnostic panel for identification of schizophrenia and provide improved classification of this disorder which is known to be comprised of overlapping subtypes (Seaton BE et al Neuropsychol Rev 2001 Mar; 11(1): 45-67).

One strong point of the present study is that samples were obtained from first onset antipsychotic naïve subjects who were well matched to their respective control populations with regards to such factors as age, gender, substance abuse and lifestyle. Almost all previous schizophrenia studies have investigated chronic patients who have been treated with antipsychotic medications, and often have multiple co-morbidities which can confound biomarker investigations. The reason for the scarcity of studies including first-onset antipsychotic naive patients is that such patients are difficult to recruit. Even large specialized centres can only recruit around 20-30 such patients each year and few centres follow strict standard operating procedures for collection of samples. In this study, patients and controls were acquired from multiple independent cohorts and underwent extensive clinical characterization. In addition, sera were collected and stored according to strict standard operating procedures to maximize reliability and accuracy of the results.

In summary, this is the first study showing a reproducible biological signature in sera of schizophrenia patients. A remarkable finding of this study was that the schizophrenia disease signature was also apparent in individuals who underwent routine blood screening in the United States military, prior to a subsequent diagnosis of schizophrenia, and before overt symptoms of mental disorder had emerged. As this signature was not apparent in other related psychiatric disorders, these findings hold promise for the future development of a rapid, specific and non-invasive blood test for schizophrenia. It should be noted that tests for disorders with a low incidence such as schizophrenia require exceptionally high specificities if used in the general population. For this reason, the most effective use of such tests would be as a confirmatory diagnostic aid by a psychiatric specialist in conjunction with a clinical assessment. In this way, the test would be used in populations already enriched for schizophrenia with the purpose of establishing and confirming a diagnosis more rapidly, as compared to the requirement for 6 months duration of continuous symptoms in a DSM IV-based diagnosis. Such an application of a biomarker test would help to initiate treatment of patients more rapidly and, therefore, reduce the duration of untreated psychosis and, in turn, improve outcomes. This would be an important breakthrough by helping clinical psychiatrists to identify vulnerable patients early in the disease process, allowing for earlier or even preventative therapeutic intervention.

## Claims

1. Use of al antitrypsin, apolipoprotein H, complement 3, carcinoembryonic antigen, cortisol, connective tissue growth factor (CTGF), ferritin, haptoglobin, interleukin-10, macrophage inflammatory factor (MIF), prolactin, serum amyloid P and tissue inhibitor of metalloprotease-1 (TIMP 1) as a specific panel of analyte biomarkers for schizophrenia or predisposition thereto.

2. A method of diagnosing or monitoring schizophrenia or predisposition thereto comprising detecting and/or quantifying, in a sample from a test subject, the specific panel of analyte biomarkers as defined in claim 1.

3. A method of monitoring efficacy of a therapy in a subject having, suspected of having, or of being predisposed to schizophrenia, comprising detecting and/or quantifying, in a sample from said subject, the specific panel of analyte biomarkers as defined in claim 1.

4. A method as defined in claim 2 or claim 3, which is conducted on samples taken on two or more occasions from a test subject.

5. A method as defined in any of claims 2 to 4, further comprising comparing the level of the biomarker present in samples taken on two or more occasions.

6. A method as defined in any of claims 2 to 5, comprising comparing the amount of the biomarker in said test sample with the amount present in one or more samples taken from said subject prior to commencement of therapy, and/or one or more samples taken from said subject at an earlier stage of therapy.

7. A method as defined in any of claims 2 to 6, further comprising detecting a change in the amount of the biomarker in samples taken on two or more occasions.

8. A method as defined in any of claims 2 to 7, comprising comparing the amount of the biomarker present in said test sample with one or more controls.

9. A method as defined in claim 8, comprising comparing the amount of the biomarker in a test sample with the amount of the biomarker present in a sample from a normal subject.

10. A method as defined in any of claims 2 to 9, wherein quantifying is performed by measuring the concentration of the analyte biomarker in the or each sample.

11. A method as defined in any of claims 2 to 10, wherein detecting and/or quantifying is performed by one or more methods selected from SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC or other LC or LC-MS-based technique.

12. A method as defined in any of claims 2 to 11, wherein detecting and/or quantifying is performed using an immunological method.

13. A method as defined in any of claims 2 to 12, wherein the detecting and/or quantifying is performed using a biosensor or a microanalytical, microengineered, microseparation or immunochromatography system.

14. A method as defined in any of claims 2 to 13, wherein the biological sample is cerebrospinal fluid, whole blood, blood serum, plasma, urine, saliva, or other bodily fluid, or breath, condensed breath, or an extract or purification therefrom, or dilution thereof.

15. Use of a kit for monitoring or diagnosing schizophrenia, comprising a biosensor capable of detecting and/or quantifying the specific panel of analyte biomarkers according to the use of claim 1.

## Patentansprüche

1. Verwendung von α1-Antitrypsin, Apolipoprotein H, Komplement 3, karzinoembryonalem Antigen, Cortisol, Bindegewebe-Wachstumsfaktor (CTGF), Ferritin, Haptoglobin, Interleukin-10, Makrophagen-Entzündungsfaktor (MIF), Prolaktin, Serum-Amyloid P und Gewebeinhibitor von Metalloprotease-1 (TIMP 1) als spezifische Gruppe von Analyt-Biomarkern für Schizophrenie oder eine Veranlagung dafür.

2. Verfahren zur Diagnose oder Überwachung von Schizophrenie oder einer Veranlagung dafür, das das Detektieren und/oder Quantifizieren der spezifischen Gruppe von Analyt-Biomarkern nach Anspruch 1 in einer Probe von einem Testsubjekt beinhaltet.

3. Verfahren zum Überwachen der Wirksamkeit einer Therapie bei einem Subjekt, das Schizophrenie hat, vermutlich Schizophrenie oder eine Veranlagung dafür hat, das das Detektieren und/oder Quantifizieren der spezifischen Gruppe von Analyt-Biomarkern nach Anspruch 1 in einer Probe von dem genannten Subjekt beinhaltet.

4. Verfahren nach Anspruch 2 oder Anspruch 3, das an Proben durchgeführt wird, die einem Testsubjekt bei zwei oder mehreren Gelegenheiten entnommen wurden.

5. Verfahren nach einem der Ansprüche 2 bis 4, das ferner das Vergleichen des Biomarkeranteils beinhaltet, der in Proben enthalten ist, die bei zwei oder mehreren Gelegenheiten entnommen wurden.

6. Verfahren nach einem der Ansprüche 2 bis 5, das das Vergleichen der Menge des Biomarkers in der genannten Testprobe mit der in einer oder mehreren Proben, die von dem genannten Subjekt vor Therapiebeginn entnommen wurden, und/oder einer oder mehreren Proben, die von dem genannten Subjekt in einem früheren Stadium der Therapie entnommen wurden, enthaltenen Menge beinhaltet.

7. Verfahren nach einem der Ansprüche 2 bis 6, das ferner das Detektieren einer Veränderung der Menge des Biomarkers in Proben beinhaltet, die bei zwei oder mehreren Gelegenheiten entnommen wurden.

8. Verfahren nach einem der Ansprüche 2 bis 7, das das Vergleichen der Menge des in der genannten Testprobe enthaltenen Biomarkers mit einer oder mehreren Kontrollen beinhaltet.

9. Verfahren nach Anspruch 8, das das Vergleichen der Menge des Biomarkers in einer Testprobe mit der Menge des in einer Probe von einem normalen Subjekt enthaltenen Biomarkers beinhaltet.

10. Verfahren nach einem der Ansprüche 2 bis 9, wobei das Quantifizieren durch Messen der Konzentration des Analyt-Biomarkers in der oder jeder Probe erfolgt.

11. Verfahren nach einem der Ansprüche 2 bis 10, wobei das Detektieren und/oder Quantifizieren mit einem oder mehreren Verfahren erfolgt, die aus SELDI (-TOF), MALDI (-TOF), einer 1D-gelbasierten Analyse, einer 2D-gelbasierten Analyse, Massenspektrometrie (MS), Umkehrphasen-(RP)-LC, Größenpermeation (Gelfiltration), Ionenaustausch, Affinität, HPLC, UPLC oder einer anderen LC- oder LC-MS-basierten Methode ausgewählt sind.

12. Verfahren nach einem der Ansprüche 2 bis 11, wobei das Detektieren und/oder Quantifizieren mit einem immunologischen Verfahren erfolgt.

13. Verfahren nach einem der Ansprüche 2 bis 12, wobei das Detektieren und/oder Quantifizieren mit einem Biosensor oder einem mikroanalytischen, mikrotechnischen, Mikrotrenn- oder Immunchromatographiesystem erfolgt.

14. Verfahren nach einem der Ansprüche 2 bis 13, wobei die biologische Probe Rückenmarksflüssigkeit, Vollblut, Blutserum, Plasma, Urin, Speichel oder eine andere Körperflüssigkeit oder Atem, kondensierter Atem oder ein Extrakt oder Reinigungsprodukt davon oder ein Verdünnungsprodukt davon ist.

15. Verwendung eines Kits zur Überwachung oder Diagnose von Schizophrenie, der einen Biosensor beinhaltet, der die spezifische Gruppe von Analyt-Biomarkern gemäß der Verwendung in Anspruch 1 detektieren und/oder quantifizieren kann.

## Revendications

1. Utilisation de l'α1-antitrypsine, de l'apolipoprotéine H, du facteur C3 du complément, de l'antigène carcino-embryonnaire, du cortisol, du facteur de croissance du tissu conjonctif (CTGF), de la ferritine, de l'haptoglobine, de l'interleukine-10, du facteur inflammatoire des macrophages (MIF), de la prolactine, du composant amyloïde P sérique et de l'inhibiteur tissulaire de la métalloprotéinase-1 (TIMP-1) en tant que batterie spécifique d'analytes biomarqueurs de la schizophrénie ou d'une prédisposition à cette affection.

2. Méthode de diagnostic ou de suivi de la schizophrénie ou d'une prédisposition à cette affection, qui comprend la détection et/ou quantification de la batterie spécifique d'analytes biomarqueurs selon la revendication 1 dans un échantillon prélevé chez un sujet testé.

3. Méthode de suivi de l'efficacité d'une thérapie chez un sujet atteint ou suspecté d'être atteint de schizophrénie ou prédisposé à cette affection, qui comprend la détection et/ou quantification de la batterie spécifique d'analytes biomarqueurs selon la revendication 1 dans un échantillon prélevé chez ledit sujet.

4. Méthode selon la revendication 2 ou la revendication 3, qui est effectuée sur des échantillons prélevés à deux occasions ou plus chez un sujet testé.

5. Méthode selon l'une quelconque des revendications 2 à 4, qui comprend également la comparaison des taux du biomarqueur présent dans des échantillons prélevés à deux occasions ou plus.

6. Méthode selon l'une quelconque des revendications 2 à 5, qui comprend la comparaison de la quantité du biomarqueur dans ledit échantillon testé à la quantité présente dans un ou plusieurs échantillons prélevés chez ledit sujet avant l'instauration de la thérapie et/ou dans un ou plusieurs échantillons prélevés chez ledit sujet à un stade plus précoce de la thérapie.

7. Méthode selon l'une quelconque des revendications 2 à 6, qui comprend également la détection d'un changement de la quantité du biomarqueur dans des échantillons prélevés à deux occasions ou plus.

8. Méthode selon l'une quelconque des revendications 2 à 7, qui comprend la comparaison de la quantité du biomarqueur présent dans ledit échantillon testé à celle mesurée dans un ou plusieurs échantillons témoins.

9. Méthode selon la revendication 8, qui comprend la comparaison de la quantité du biomarqueur dans un échantillon testé à la quantité du biomarqueur présent dans un échantillon prélevé chez un sujet normal.

10. Méthode selon l'une quelconque des revendications 2 à 9, où la quantification est effectuée en mesurant la concentration en l'analyte biomarqueur dans l'échantillon ou dans chaque échantillon.

11. Méthode selon l'une quelconque des revendications 2 à 10, où la détection et/ou quantification est effectuée par une ou plusieurs techniques sélectionnées parmi les suivantes : SELDI (-TOF), MALDI (-TOF), analyse sur gel 1-D, analyse sur gel 2-D, spectrométrie de masse (SM), CL en phase inverse (PI), chromatographie par perméation de gel (filtration sur gel), chromatographie par échange d'ions, chromatographie d'affinité, CLHP, CLUP ou toute autre technique reposant sur la CL ou la CL-SM.

12. Méthode selon l'une quelconque des revendications 2 à 11, où la détection et/ou quantification est effectuée en utilisant une méthode immunologique.

13. Méthode selon l'une quelconque des revendications 2 à 12, où la détection et/ou quantification est effectuée en utilisant un biocapteur ou un système de microanalyse, de micromécanique, de microséparation ou d'immunochromatographie.

14. Méthode selon l'une quelconque des revendications 2 à 13, où l'échantillon biologique est le liquide céphalorachidien, le sang entier, le sérum sanguin, le plasma, l'urine, la salive ou tout autre liquide corporel ou l'haleine, l'haleine condensée ou un extrait ou produit de purification ou dilution de ceux-ci.

15. Utilisation d'un kit pour le suivi ou le diagnostic de la schizophrénie, qui comprend un biocapteur capable de détecter et/ou de quantifier la batterie spécifique d'analytes biomarqueurs selon l'utilisation de la revendication 1.
